(19) Europäisches Patentamt
European Patent Office
Office européen des brevets

(11) EP 4 239 043 A1

(12) EUROPEAN PATENT APPLICATION

(43) Date of publication:
06.09.2023 Bulletin 2023/36

(21) Application number: 22382185.1

(22) Date of filing: 01.03.2022

(51) International Patent Classification (IPC):
*C11D 1/62* (2006.01) *C11D 1/835* (2006.01)
*C11D 3/00* (2006.01) *C11D 3/50* (2006.01)
*C11D 11/00* (2006.01) *A61Q 5/12* (2006.01)
*C07C 209/20* (2006.01) *C11D 17/04* (2006.01)
*C11D 1/74* (2006.01)

(52) Cooperative Patent Classification (CPC):
C11D 1/62; A61Q 5/12; C11D 1/835; C11D 3/0015;
C11D 3/50; C11D 11/0017; C11D 17/043;
C11D 1/74

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: Kao Corporation S.A.U
08210 Barberà del Vallès Barcelona (ES)

(72) Inventors:
- Pi Boleda, Bernat
  08210 Barberà del Vallès (Barcelona) (ES)
- Sobrevias Alabau, Jaume
  08210 Barberà del Vallès (Barcelona) (ES)
- Nogués López, Blanca
  08210 Barberà del Vallès (Barcelona) (ES)
- Mundó Blanch, Miquel
  08210 Barberà del Vallès (Barcelona) (ES)

(74) Representative: Hoffmann Eitle
Patent- und Rechtsanwälte PartmbB
Arabellastraße 30
81925 München (DE)

# (54) DISPERSIBLE SOFTENER FORMULATIONS AND UNIT DOSES

(57) The present invention generally relates to mixtures of cationic surfactants useful for preparing concentrated softening compositions that can be introduced directly to the washing machine as a unit dose (e.g., tablet or capsule) or as a concentrated product; or can be used to prepare a fabric softener at home using tap water; and their use for softening fabrics and/or fibres.



Processed by Luminess, 75001 PARIS (FR)

## Description

### FIELD OF THE INVENTION

The present invention generally relates to mixtures of cationic surfactants useful for preparing concentrated softening compositions that can be introduced directly to the washing machine as a unit dose (e.g., tablet or capsule) or as a concentrated product; or can be used to prepare a fabric softener at home using tap water; and their use for softening fabrics and/or fibres.

### BACKGROUND OF THE INVENTION

Quaternary ester ammonium compounds, commonly referred to as "esterquats" (EQ), have found broad use as fabric softener actives due to their high softening performance (e.g., for softening textile fibers and fabrics as well as keratinous fibers, such as hair), their biodegradability, reasonably low aquatic toxicity, and good cosmetic compatibility with the skin.

Softener formulations comprising esterquats have the disadvantage of having limited compatibility with perfumes, especially in concentrated products. However, there is a demand for softener formulations comprising a softening agent in concentrated form, which, in combination with perfume, show a clear viscous liquid that can be directly dispersed in water. Additionally, it is desirable that such formulations are stable upon storage.

While there are attempts in the state of the art aiming at related objectives, there remains a demand for surfactant mixtures capable of providing softening formulations meeting the above requirements

In WO2016096614A1, a fabric treatment agent comprising specific esterquats is described. However, the additional presence of cationic thickeners and non-ionic emulsifiers is required to yield an optically clear, viscous fabric softener formulation with high storage stability.

In view of the above, the present invention aims at the problem of providing new cationic surfactant mixtures allowing to prepare clear, viscous and stable concentrated softener compositions, even in the absence of further additives. In particular, the present invention aims at providing such mixtures which, in combination with perfume, result in a clear viscous liquid that can be introduced directly to the washing machine as a unit dose (e.g., tablet or capsule) or as a concentrated product; or can be used to prepare a fabric softener at home using tap water. Additionally, it is preferable that such mixtures and compositions exhibit advantageous storage stability under various conditions, advantageous handleability (simple handling), and/or advantageous softening properties.

### SUMMARY OF THE INVENTION

As a solution to the above problems, the present invention provides a mixture of cationic surfactants is obtainable from a process comprising the steps: Step I: esterification of a) with b), and Step II: cation formation from the reaction products of Step I, wherein:

a) is a hydroxyl group-containing compound or a mixture of hydroxyl group-containing compounds comprising a.1 and optionally a.2, wherein:

- a.1 is an alkanolamine or a mixture of alkanolamines of the general formula (I):

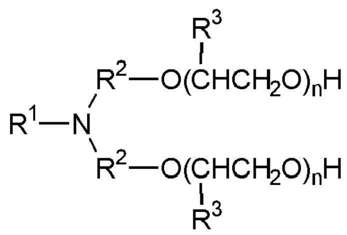

**(I)**

in which $R^1$ is selected from hydrogen, a $C_1$-$C_6$ alkyl group, and the residue

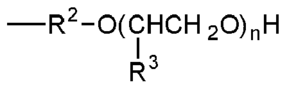

.

$R^2$ is a $C_1$-$C_6$ alkylene group, $R^3$ is hydrogen or methyl, n is 0 or an integer from 1 to 20; and

- a.2 is a polyol, which can be optionally alkoxylated, and is characterized by a MW in the range 60 to 190 g/mol;

b) is a mixture of compounds containing one or more carboxylic groups comprising b.1 and b.2, wherein:

- b.1 is a monocarboxylic acid or a mixture of monocarboxylic acids of formula (II):

$R^6$-COOH (II)

in which $R^6$ is a linear or branched $C_6$-$C_{23}$ alkyl or alkenyl group; or an alkyl ester or glyceride thereof, preferably a linear or branched $C_6$-$C_{23}$ alkyl or alkenyl ester; and

- b.2 is a dicarboxylic acid or a mixture dicarboxylic acids of the general formula (III), or reactive derivative(s) thereof:

HOOC-L-COOH (III)

wherein L is a saturated or unsaturated, linear, branched or cyclic group having 1 to 10 carbon atoms, each of which carbon atoms is optionally substituted by a $C_1$-$C_6$ saturated or unsaturated group; and is preferably represented by $(CH(R^7))_m$ or by ($C_6$-$C_{10}$ arylene) optionally substituted by one or more $R^7$, in which each $R^7$ is independently a hydrogen, OH or a $C_1$-$C_6$ saturated or unsaturated group, m is 0 or an integer from 1 to 10, wherein for $m \geq 2$, the chain $(CH)_m$ optionally contains one or more double bonds and/or cyclic group(s);

wherein a.1), a.2), b.1) and b.2) are introduced in the reaction system of Step I in amounts resulting in the following molar ratios:

- the molar ratio of monoacid(s)/diacid(s) (b.1/b.2) is 0.10 to 5.0;
- the molar ratio between organic carboxylic groups and organic hydroxyl groups (COOH/OH) present in the system is 0.30 to 0.70; and
- the molar ratio between the compound(s) within the definition a.2 and the compound/s under definition a.1 is 0 to 0.5.

The present invention further provides compositions comprising the above mixture, and in particular concentrated compositions comprising the above mixture and preferably a perfume, which are useful for softening fabrics or fibres, e.g., by comprising mixing the composition with tap water, or by introducing the concentrated composition directly or in the form of a unit dose into a washing machine.

## DETAILED DESCRIPTION OF THE INVENTION

The present inventors have surprisingly found that particular mixtures of cationic surfactants (esterquats (EQ)) obtainable by reacting a combination of mono- and di-carboxylic acids with alkanolamine(s) and optionally polyol(s), and subsequent cation formation, can form, in combination with perfume, clear and stable viscous liquids that can be introduced directly to the washing machine as a tablet or a concentrated product, or can be used to prepare a fabric softener at home using tap water. These effects can be observed even in the absence of further additives.

In general, the cationic surfactant mixtures show good compatibility with perfume. Such combined, concentrated surfactant or surfactant/perfume comprising compositions are clear and homogeneous at room temperature, and show good stability upon storage at room temperature, and preferably also under various other conditions (5 °C, 40 °C).

The concentrated softener compositions have multiple advantageous applications,

For instance, they can be added directly to the washing machine to provide softening effect. They can provide fast dispersion in water, and have the advantage of no need to stabilize diluted formulations. Alternatively, the concentrated softener compositions can be used to formulate fabric softeners at home using tap water. They can provide fast dispersion in water, and no need to stabilize diluted formulations

Yet further, concentrated softener compositions can be useful as an appealing, optically attractive and advantageous fabric softening component of unit doses (e.g., tablets or capsules) which can be directly introduced into a washing machine by the consumer. Due to the good compatibility of the cationic surfactant mixture with perfume, it is possible to provide the fabric softening component in highly concentrated form, which in turns allows for preparing unit doses of small and easy to handle size.

Overall, the mixtures, compositions and unit doses of the invention can contribute to various advantageous effects, such as water reduction, plastic reduction, energy reduction, ecological acceptability and/or sustainability of the products and their use.

***Mixtures of cationic surfactants***

The cationic surfactant mixtures of the present invention can be mixed with perfume, thereby providing softener compositions that are clear, stable and viscous. No additives are necessary for achieving the optical transparency and stability of the final compositions. Transparency and viscosity are attractive and desirable features of the products. Using less adititives is advantageoous from of environmental, bio- and skin compatibility, and economical perspective.

In an embodiment of the invention, the molar ratio of monoacid(s)/diacid(s) (b.1/b.2) is 0.5 to 3.5, preferably 1.5 to 3.5; and/or the molar ratio between organic carboxylic groups and organic hydroxyl groups (COOH/OH) present in the system is 0.40 to 0.70, preferably 0.50 to 0.70; and/or the molar ratio between the compound(s) within the definition a.2 and the compound/s under definition a.1 is 0 or 0.1 to 0.5, preferably 0.1 to 0.5. Without wishing to be bound by theory, these particular ratios individually or in combination can contribute to even further improving the softening effect and/or the stability of the formulations formed by using the mixtures of cationic surfactants according to the present invention.

Particularly good stability is achieved when the molar ratio between organic carboxylic groups and organic hydroxyl groups (COOH/OH) in the definition of the mixture of cationic surfactant below 0.7.

In an embodiment of the invention, the molar ratio of monoacid(s)/diacid(s) (b.1/b.2) is 0.10 to 1.0, preferably 0.20 to 1.0, more preferably 0.30 to 1.0; and/orthe molar ratio between organic carboxylic groups and organic hydroxyl groups (COOH/OH) present in the system is 0.30 to 0.70, preferably 0.40 to 0.70, more preferably 0.50 to 0.70; and/or the molar ratio between the compound(s) within the definition a.2 and the compound/s under definition a.1 is 0 or 0.1 to 0.5, preferably 0.1 to 0.5. Without wishing to be bound by theory, each of these particular ratios can contribute to improving the optical transparency. Additionally, handleability (simple handling), and/or softening properties may be improved. Particularly good transparency is achieved at a water content lower than 5%, preferably 0-5% w/w, most preferably in the absence or essential absence of water.

In an embodiment of the invention, the alkanolamine(s) of formula (I) is/are selected from triethanolamine, N-methyldiethanolamine, N-methyldiisopropanolamine and triisopropanolamine, each of which is optionally alkoxylated with ethylene oxide or propylene oxide, and mixtures thereof.

In an embodiment of the invention, in the dicarboxylic acid(s) of formula (III), each L is selected from ethane-1,2-diyl, 1-hydroxyethane-1,2-diyl, *cis*-ethene-1,2-diyl, *trans*-ethene-1,2-diyl, propane-1,2-diyl, propane-1,3-diyl, butane-1,4-diyl, pentane-1,5-diyl, hexane-1,6-diyl, cyclohexane-1,4-diyl, octane-1,8-diyl and 1,4-phenylenyl; preferably butane-1,4-diyl, hexane-1,6-diyl or octane-1,8-diyl.

In an embodiment of the invention, the dicarboxylic acid of formula (III) is selected from succinic, malic, glutaric, adipic, sebacic, pimelic, suberic, maleic and terephthalic acid, acids obtained by thermal oligomerisation of unsaturated fatty acids, and mixtures thereof.

In an embodiment of the invention, the reactive derivative(s) of the dicarboxylic acid(s) of the general formula (III) are one or more selected from halide, anhydride, preferably mixed anhydride with acetic acid or cyclic anhydride.

The inventors have surprisingly found out that by using the particular ratios of the components as in the present invention, it is possible to obtain cationic surfactant mixtures allowing to prepare clear and stable softener formulations when mixed with water at room temperature, even when using a broader variety of monocarboxylic acid(s) than in the prior art.

For example, the monocarboxylic acid(s) of formula (II) are synthetic fatty acids and/or are obtained from fats or oils of natural origin, and are optionally hydrogenated; or are derived from oils of vegetal origin which are optionally hydrogenated.

In an embodiment of the invention, the monocarboxylic acid(s) of formula (II) are selected from those which are obtained from tallow, palm, olive, coconut, sunflower, soya, rapeseed, grape marc and grape, each of which can be hydrogenated, partially hydrogenated, or non-hydrogenated.

In an embodiment of the invention, the iodine value of the carboxylic monoacid(s) of formula (II) is preferably $\geq 5$, more preferably $\geq 35$, even more preferably $\geq 45$, most preferably $\geq 50$; and/or preferably $\leq 280$, more preferably $\leq 180$, even more preferably $\leq 100$, most preferably $\leq 80$. Preferably, the iodine value of the carboxylic monoacid(s) of formula (II) is 5-280, more preferably 5-180, more preferably 35-180, more preferably 45-100, more preferably 45-80, most preferably 50-80. Without wishing to be bound by theory, using carboxylic monoacid(s) having such iodine values can contribute to even further improving the optical transparency at various temperatures, and/or the storage stability of the aqueous formulations obtained from the mixture of cationic surfactants according to the present invention.

In an embodiment of the invention, the carboxylic monoacid(s) of formula (II) is one or more selected from caproic acid, caprylic acid, 2-ethylhexanoic acid, capric acid, lauric acid, isotridecanoic acid, myristic acid, palmitic acid, palmoleic acid, stearic acid, isostearic acid, oleic acid, elaidic acid, petroselinic acid, linoleic acid, linolenic acid, eleostearic acid, arachic acid, gadoleic acid, behenic acid and erucic acid, and mixtures thereof which are obtained for example by pressure splitting of natural fats and oils, in the reduction of aldehydes from Roelen's oxosynthesis or dimerization of unsaturated fatty acids, stearic acids, isostearic acid, palmitic acid, myristic acid, lauric acid, capric acid, caprylic acid,

2-ethylhexanoic acid, 2-octyldodecanoic acid, capric acid, oleic acid, linoleic acid, linolenic acid, partially hydrogenated coconut fatty acid, palm fatty acid, partially hydrogenated distilled palm fatty acid, hydrogenated distilled palm fatty acid, palm kernel fatty acid, tallow fatty acid, distilled tallow fatty acid, and rapeseed fatty acid.

In an embodiment of the invention, the compounds corresponding to a.1 and/or a.2 can be from natural origin or from synthetic origin.

In an embodiment of the invention, the polyol a.2 is one or more selected from trimethylolpropane (TMP), glycerine and neopentyl glycol (NPG), each of which can be optionally alkoxylated, preferably ethoxylated; wherein the polyol a.2 is more preferably trimethylolpropane (TMP), or is absent.

Step I is and esterification step of reacting a) with b). In an exemplary embodiment, monoacid b.1 and diacid b.2 are combined with alkanolamine a.1 and optionally the polyol b.2. The obtained mixture is heated. Preferably, the mixture is heated to reflux under atmospheric pressure, e.g., for 1-5, preferably 2-4 hours at 140-200 °C, preferably 160-180°C. Preferably, step I is performed until no more water is distilled off the reaction mixture.

The reaction product obtained from step I is subjected to cation formation in step II. Preferably, an organic solvent is added before step II. Step II can correspond to the formation of the addition salts of the alkanolamine esters obtained from Step I with mineral or organic acids, preferably wherein the mineral or organic acids are one or more selected from hydrochloric, sulphuric, phosphoric, citric and lactic acid. Alternatively, Step II can correspond to the quaternisation of reaction mixtures of Step I with alkylating agent(s), preferably wherein the alkylating agents are one or more selected from methyl chloride, methyl bromide, dimethyl sulphate, diethyl sulphate and dimethyl carbonate. Step II can be performed at room temperature or elevated temperature, e.g., 40-100 °C, preferably 50-90 °C; preferably for 1-5, more preferably 2-4 hours, or until the virtually complete absence of amine value was verified by acid/base assay.

In an embodiment of the invention, the mixture further comprises an organic solvent, preferably an alcohol, more preferably ethanol, n-propanol or isopropanol, butanols, glycol, propane or butanediol, glycerol, diglycol, propyl or butyl diglycol, ethylene glycol methyl ether, ethylene glycol ethyl ether, ethylene glycol propyl ether, ethylene glycol mono-n-butyl ether, diethylene glycol methyl ether, diethylene glycol ethyl ether, propylene glycol methyl, ethyl or propyl ether, dipropylene glycol methyl or ethyl ether, methoxy, ethoxy or butoxy triglycol, 1-butoxyethoxy-2-propanol, 3-methyl-3-methoxybutanol, or propylene glycol t-butyl ether. For instance, such solvent may be added during the preparation step, e.g., during Step I and/or II, preferably before Step II.

In an embodiment of the invention, the content of the organic solvent in the cationic surfactant mixture is 0-30%, preferably 0-20%, more preferably 10-20% by weight.

In an embodiment of the invention, the mixture is essentially water-free.

In an embodiment of the invention, the mixture essentially consists of the reaction products of steps I and II, and optionally an organic solvent. Preferably, the mixture consists of the reaction products of steps I and II and solvent, if any, and unreacted starting materials as well as inevitable impurities from the production process, if any.

## Softener *compositions*

Further provided is a softener composition, preferably a fabric-softening and/or keratin-based-fibres-softening composition, comprising the cationic surfactant mixture according to the invention. The composition preferably further comprises water.

Such compositions exhibit can exhibit advantageous storage stability under various conditions, as well as further advantageous properties, such as handleability (simple handling) and/or softening properties.

Such compositions may be clear solutions at RT, and can be stable upon storage RT, or 5°C, or at 40°C, e.g., for at least 2 months. Particularly good stability is achieved when the molar ratio between organic carboxylic groups and organic hydroxyl groups (COOH/OH) in the definition of the mixture of cationic surfactant is 0.7, more preferably below 0.7. In an embodiment of the invention, the composition may be optically transparent at room temperature, and preferably also at temperatures of 26-40 °C, more preferably 20-50 °C, even more preferably 16-50 °C, most preferably 5-60 °C. Particularly good optical transparency is achieved when the molar ratio of monoacid(s)/diacid(s) (b.1/b.2) is 1.0 or below.

In an embodiment of the invention, the composition further comprises a perfume. The perfume consists of one or more substance(s). The average logP of the perfume substance(s) is from 1 to 6. The weight ratio between the cationic surfactant mixture according to the invention, as defined hereinabove, and the perfume is from 99:1 to 40:60, more preferably from 80:20 to 60:40, even more preferably 80:20 to 70:30.

Preferably, the water content in the compositions of the invention is 80% or less or 40% or less, more preferably 35% or less, most preferably 30% w/w or less. The solid residue is preferably 60% or more, more preferably 65% or more, even more preferably between 65 and 70%, most preferably 70% or more. In an alternative embodiment, the water content in the compositions is preferably higher than 40%, more preferably higher than 80%, most preferably higher than 85% w/w. The solid residue is preferably lower than 50%, more preferably lower than 25%, even more preferably between 15 and 2%.

In an embodiment of the invention, the composition further comprises a non-ionic surfactant. The weight ratio

between the cationic surfactant mixture and the non-ionic surfactant is preferably from 100:0 (i.e., no non-ionic surfactant is present) to 70:30. A non-ionic surfactant can advantageously improve solubility of a perfume in the composition, especially when high amounts of perfume are used (e.g., e.g., > 1%, optionally up to 3% by weight of the composition, or more). Preferably, when the amount of perfume in the composition is 0.3% by weight or less, no non-ionic surfactant is used; otherwise, a non-ionic surfactant is optionally used. Additionally, the addition of a non-ionic surfactant can even further improve the good stability of the formulation upon storage.

In an embodiment of the invention, the composition further comprises a thickener, e.g., a thickening polymer. The weight ratio the cationic surfactant mixture to the thickener is preferably from 150:1 to 10:5, more preferably from 100:1 to 10:2. A thickener may be added to increase the viscosity of the final composition. Suitable thickeners are, e.g., PEG-150 distearate, Hydroxyethyl cellulose, hydroxymethyl cellulose and derivatives thereof, PEG-120 Methyl Glucose Dioleate, PEG-120 Methyl Glucose Trioleate (and) propanediol, ethoxylated Sorbitan Triisostearate (e.g., PEG-160 Sorbitan Triisostearate, such as Kaopan TWIS-559S from Kao Chemicals Europe, S.L.), and copolymers of acrylamide and dimethyl amino ethyl methacrylate methyl chloride cross- methylene bisacrylamide (such as FLOSOFT 222 manufactured by SNF).

In an embodiment of the invention, the composition comprises: (i) the mixture of cationic surfactants as described hereinabove, and (ii) optionally one or more non-ionic surfactants, wherein the sum of components (i) and (ii) is from 2% to 100% by weight, and wherein:

- the content of component (i) is from 2% to 100%, relative to the weight of the composition; and
- the content of component (ii) is from 0% to 50%, preferably from 0 to 20%; more preferably from 0 to 5%, even more preferably 0 to 2%, most preferably 0 to 0.05% by weight, relative to the weight of the composition.

Preferably, for diluted formulations:

- the sum of components (i) and (ii) is from 2% to 50% by weight,
- the content of component (i) is from 2% to 50%,
- the content of component (ii) is from 0% to 15%, preferably from 0 to 10%; more preferably from 0 to 5%, even more preferably 0 to 2%, most preferably 0 to 0.05% by weight, relative to the weight of the composition.

Preferably, for water-free formulations: the sum of components (i) and (ii) is from 50% to 100% by weight, wherein,

- the content of component (i) is from 50% to 100%,
- the content of component (ii) is from 0% to 50%, preferably from 0 to 30%; more preferably from 0 to 20%, even more preferably 0 to 10%, most preferably 0 to 5% by weight, relative to the weight of the composition

Preferred embodiments of compositions according to the invention comprise, preferably essentially consist, more preferably consist of: esterquats (EQ) and water; or EQ, water and thickener; or EQ, water and up to 3.0% by weight of perfume; or EQ, water, non-ionic surfactant(s) and perfume; or EQ, water, thickener, and perfume; or EQ, organic solvent, such as ethanol or glycerine, and perfume.

Preferred embodiments of water-free compositions according to the invention comprise, preferably essentially consist, more preferably consist of: esterquats (EQ); or EQ and thickener; or EQ and perfume; or EQ, non-ionic surfactant(s) and perfume; or EQ, thickener, and perfume; or EQ, organic solvent, such as ethanol or glycerine, and perfume.

***Compositions suitable for preparing a unit dose softener***

The present invention further provides a concentrated composition suitable for preparing a unit dose softener contained in a water-soluble pouch, wherein the composition comprises a mixture of cationic surfactants as described hereinabove, and is liquid and transparent at a temperature from 26-40 °C, preferably 20-60 °C, more preferably 16-80 °C, most preferably 0-80 °C. The concentrated composition preferably has a water content lower than 10%, preferably lower than 5%, preferably lower than 1%, preferably lower than 0.1%, most preferably 0%.

Preferably, the concentrated composition suitable for preparing a unit dose contains 40% or more, more preferably 40-70%, most preferably about 70% w/w of the cationic surfactant mixture (EQ).

Such compositions may contain a perfume and/or a non-ionic surfactant. The perfume preferably consists of one or more substance(s). The average logP of the perfume substance(s) is from 1 to 6.

Particularly good stability of the compositions comprising the mixture of cationic surfactants and perfume is achieved when the molar ratio between organic carboxylic groups and organic hydroxyl groups (COOH/OH) in the definition of the mixture of cationic surfactants is below 0.7.

Preferably, the weight ratio of the described components are values according to the following ratios:

P:C is a value from 0:100 to 60:40
NI:C is 0 or a value from 0:100 to 50:50
P corresponds to the weight content of perfume,
C corresponds to the weight content of cationic surfactant mixture according to claims 1 to 4,
NI corresponds to the weight content of non-ionic surfactant.

Preferably, such concentrated compositions essentially consist, or consist of, the mixture of cationic surfactants as described hereinabove, perfume, optionally non-ionic surfactant, and optionally an organic solvent.

***Unit dose softeners***

The present invention further provides a fabric conditioner unit dose (i.e., a unit dose softener) comprising: a water-soluble pouch acting as container of a liquid composition; and the concentrated composition suitable for preparing a unit dose, as described hereinabove, contained within or by the pouch. Preferably, the unit dose is a capsule or a tablet. Preferably, the water-soluble pouch is formed of or comprises polyvinyl alcohol.

***Concentrated compositions suitable for being dispersed in cold water by consumer at home***

The present invention further provides a concentrated softener composition comprising the cationic surfactant mixture as described hereinabove, and having a water content of 80% or lower, preferably 50% or lower, more preferably 30% or lower, even more preferably lower than 5% w/w , most preferably lower than 1% w/w. Such composition, e.g., having lower than 5% w/w water, can be liquid and transparent at temperature from 26-40 °C, preferably 20-60 °C, more preferably 16-60 °C, most preferably 0-80 °C.

Preferably, the concentrated composition contains 40% or more, more preferably 40-70, most preferably about 70% w/w of the cationic surfactant mixture (EQ).

The present invention further provides a concentrated softener composition suitable for preparing a domestic softener formulation by dilution. The composition is liquid at temperature from 26-40 °C, preferably 20-60 °C, more preferably 16-80 °C, most preferably 0-80 °C; comprises the cationic surfactant mixture as described hereinabove, and has a water content lower than 50%, preferably lower than 30%, preferably lower than 5%.

In an embodiment of the invention, such concentrated softener composition further comprises:

A) a perfume, preferably wherein:

(i) the perfume consists of one or more substance(s); and/or
(ii) the average logP of the perfume substance(s) is from 1 to 6; and/or
(iii) the perfume is an encapsulated perfume, preferably the perfume is encapsulated in a biodegradable microcapsule, more preferably the microcapsule is based on chitosan; and/or

B) a non-ionic surfactant,

and is preferably characterised in that the weight ratio of the described components are values according to the following ratios:

P:C is a value from 0:100 to 60:40 NI:C is from 0:100 to 30:70
P corresponds to the perfume, optionally microencapsulated perfume;
C corresponds to the cationic surfactant mixture;
NI corresponds to the non-ionic surfactant.

Particularly good stability of the compositions comprising the mixture of cationic surfactants and perfume is achieved when the molar ratio between organic carboxylic groups and organic hydroxyl groups (COOH/OH) in the definition of the mixture of cationic surfactants is below 0.7.

As used herein, the non-ionic surfactant(s) can be selected from fatty acids, linear or branched, alkoxylated or non-alkoxylated esters of fatty acids, especially those containing from 8 to 18 carbon atoms, alkoxylated or non-alkoxylated Guerbet alcohols, optionally alkoxylated glycerol and polyglycerol esters, xylitol esters, alkoxylated or non-alkoxylated sorbitan esters, esters of sugars, such as glucose, fructose, galactose, mannose, xylose, arabinose, ribose, 2-deoxyribose and sucrose, $C_{8-18}$ fatty alcohols, alkyl polyglucosides, non-ionic surfactants with amide groups derived

from amines, such as glucamine, and the derivatives of methylethanolamine, diethanolamine, isopropanolamine and monoethanolamine, with linear or branched fatty acids, especially those containing from 8 to 18 carbon atoms, waxes, such as paraffins, microcrystalline waxes derived from petroleum, and synthetic waxes, and pentaerythritol esters, especially having a tallow, hydrogenated tallow, palm, behenic or oleic chain, preferably non-ionic surfactants are selected from glycerine esters that are ethoxylated, sorbitan monoesters and pentaerythritol esters, especially those having a tallow, hydrogenated tallow, palm, behenic or oleic chain.

Suitable non-ionic surfactants are Glycereth-6 Cocoate (e.g., Levenol F-200), Glycereth-17 Cocoate (e.g., Levenol C-201), ethoxylated $C_{13-15}$ alcohol (e.g., with 7EO, such as Findet 1315/19), ethoxylated $C_{16-18}$ alcohol (e.g., with 23 EO, such as Findet 1618/35); particularly suitable is ethoxylated hydrogenated castor oil (e.g., with 40 EO, such as Findet ARH-52).

Nevertheless, in any of the compositions described herein, addition of non-ionic surfactants, thickeners and organic solvents is optional, and not required for achieving the advantageous properties of the compositions, such as perfume compatibility, dispersibility, viscosity and/or stability. That is, non-ionic surfactants, thickeners and/or organic solvents may be absent.

The compositions described above may have a viscosity at 20 °C of 200-50,000 cps, as measured on a Brookfield LVT viscometer with spindle 2 at 60 rpm or with spindle 4 at 12 rpm. Preferably, such compositions have a viscosity of 200 to 5,000 mPas as measured on a Brookfield LVT viscometer with spindle 4 at 12 rpm, optionally 200 to 800 mPas; or have a viscosity of 200 to 800 mPas as measured on a Brookfield LVT viscometer with spindle 2 at 30 rpm.

For example, concentrated EQ and their mixtures with NI, perfume and/or glycols can have a viscosity values between 100 and 50000 mPa·s. Diluted formulations (from 2% active to 50% active) can have viscosity values between 2 and 500 mPa·s.

***Uses***

The present invention further provides the use of any of the compositions or unit doses described herein for softening fabrics and/or keratin-based-fibres.

The present invention further provides the use of the concentrated composition to prepare a fabric softener at home using tap water. The ready-to-use diluted fabric softener obtained thereby can be used for softening fabrics and/or keratin-based-fibres.

The present invention further provides the use the unit dose of claim or the concentrated composition for softening fabrics and/or keratin-based-fibres, comprising introducing the unit dose or the composition directly into a washing machine.

The present invention also provides the following aspects.

1. A mixture of cationic surfactants obtainable from a process comprising the steps:

Step I: esterification of a) with b), and
Step II: cation formation from the reaction products of Step I,
wherein:

a) is a hydroxyl group-containing compound or a mixture of hydroxyl group-containing compounds comprising a.1 and optionally a.2, wherein:

- a.1 is an alkanolamine or a mixture of alkanolamines of the general formula (I):

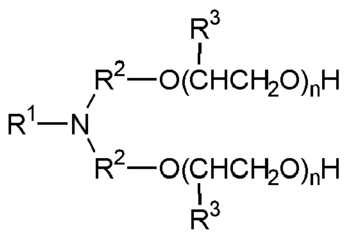

(I)

in which $R^1$ is selected from hydrogen, a $C_1$-$C_6$ alkyl group, and the residue

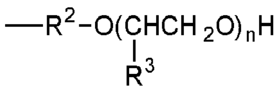

, $R^2$ is a $C_1$-$C_6$ alkylene group, $R^3$ is hydrogen or methyl, n is 0 or an integer from 1 to 20; and

- a.2 is a polyol, which can be optionally alkoxylated, and is characterized by a MW in the range 60 to 190 g/mol;

b) is a mixture of compounds containing one or more carboxylic groups comprising b.1 and b.2, wherein:

- b.1 is a monocarboxylic acid or a mixture of monocarboxylic acids of formula (II):

$$R^6\text{-COOH} \qquad (II)$$

in which $R^6$ is a linear or branched $C_6$-$C_{23}$ alkyl or alkenyl group; or an alkyl ester or glyceride thereof, preferably a linear or branched $C_6$-$C_{23}$ alkyl or alkenyl ester; and

- b.2 is a dicarboxylic acid or a mixture dicarboxylic acids of the general formula (III), or reactive derivative(s) thereof:

$$\text{HOOC-L-COOH} \qquad (III)$$

wherein L is a saturated or unsaturated, linear, branched or cyclic group having 1 to 10 carbon atoms, each of which carbon atoms is optionally substituted by a $C_1$-$C_6$ saturated or unsaturated group; and is preferably represented by $(CH(R^7))_m$ or by ($C_6$-$C_{10}$ arylene) optionally substituted by one or more $R^7$, in which each $R^7$ is independently a hydrogen, OH or a $C_1$-$C_6$ saturated or unsaturated group, m is 0 or an integer from 1 to 10, wherein for $m \geq 2$, the chain $(CH)_m$ optionally contains one or more double bonds and/or cyclic group(s);

wherein a.1), a.2), b.1) and b.2) are introduced in the reaction system of Step I in amounts resulting in the following molar ratios:

- the molar ratio of monoacid(s)/diacid(s) (b.1/b.2) is 0.10 to 5.0;
- the molar ratio between organic carboxylic groups and organic hydroxyl groups (COOH/OH) present in the system is 0.30 to 0.70; and
- the molar ratio between the compound(s) within the definition a.2 and the compound/s under definition a.1 is 0 to 0.5.

2. The mixture according to aspect 1, wherein the molar ratio of monoacid(s)/diacid(s) (b.1/b.2) is 0.5 to 3.5, preferably 1.5 to 3.5.

3. The mixture according to aspect 1 or 2, wherein the molar ratio between organic carboxylic groups and organic hydroxyl groups (COOH/OH) present in the system is 0.40 to 0.70, preferably 0.50 to 0.70

4. The mixture according to any one of the preceding aspects, wherein the molar ratio between the compound(s) within the definition a.2 and the compound/s under definition a.1 is 0 or 0.1 to 0.5, preferably 0.1 to 0.5.

5. The mixture according to any one of the preceding aspects, the amounts of the compounds a.1, a.2, b.1 and b.2 are introduced in the reaction system of Step I in amounts that result in the following molar ratios:

A)

- the molar ratio of monoacid(s)/diacid(s) (b.1/b.2) is 1.5 to 3.5;
- the molar ratio between organic carboxylic groups and organic hydroxyl groups (COOH/OH) present in the system is 0.40 to 0.70; and
- the molar ratio between the compound(s) within the definition a.2 and the compound/s under definition a.1 is 0, or is 0.1 to 0.5; or

B)

- the molar ratio of monoacid(s)/diacid(s) (b.1/b.2) is 0.10 to 1.0, preferably 0.20 to 1.0, more preferably 0.30 to 1.0; and/or
- the molar ratio between organic carboxylic groups and organic hydroxyl groups (COOH/OH) present in the system is 0.30 to 0.70, preferably 0.40 to 0.70, more preferably 0.50 to 0.70; and/or
- the molar ratio between the compound(s) within the definition a.2 and the compound/s under definition a.1 is 0 or 0.1 to 0.5, preferably 0.1 to 0.5.

6. The mixture according to any one of the preceding aspects, wherein the alkanolamine(s) of formula (I) is/are selected from triethanolamine, N-methyldiethanolamine, N-methyldiisopropanolamine and triisopropanolamine, each of which is optionally alkoxylated with ethylene oxide or propylene oxide, and mixtures thereof.

7. The mixture according to any one of the preceding aspects, wherein in the dicarboxylic acid(s) of formula (III), each L is selected from ethane-1,2-diyl, 1-hydroxyethane-1,2-diyl, *cis*-ethene-1,2-diyl, *trans*-ethene-1,2-diyl, propane-1,2-diyl, propane-1,3-diyl, butane-1,4-diyl, pentane-1,5-diyl, hexane-1,6-diyl, cyclohexane-1,4-diyl, octane-1,8-diyl and 1,4-phenylenyl; preferably butane-1,4-diyl, hexane-1,6-diyl or octane-1,8-diyl.

8. The mixture according to any one of the preceding aspects, wherein the dicarboxylic acid of formula (III) is selected from succinic, malic, glutaric, adipic, sebacic, pimelic, suberic, maleic and terephthalic acid, acids obtained by thermal oligomerisation of unsaturated fatty acids, and mixtures thereof.

9. The mixture according to any one of the preceding claims, wherein the reactive derivative(s) of the dicarboxylic acid(s) of the general formula (III) are one or more selected from halide, anhydride, preferably mixed anhydride with acetic acid or cyclic anhydride.

10. The mixture according to any one of the preceding aspects, wherein the monocarboxylic acid(s) of formula (II) are synthetic fatty acids and/or are obtained from fats or oils of natural origin, and are optionally hydrogenated.

11. The mixture according to any one of the preceding aspects, wherein the monocarboxylic acid(s) of formula (II) are derived from oils of vegetal origin which are optionally hydrogenated.

12. The mixture according to any one of the preceding aspects, wherein the monocarboxylic acid(s) of formula (II) are selected from those which are obtained from tallow, palm, olive, coconut, sunflower, soya, rapeseed, grape marc and grape, each of which can be hydrogenated, partially hydrogenated, or non-hydrogenated.

13. The mixture according to any one of the preceding aspects, wherein the iodine value of the carboxylic monoacid(s) of formula (II) is preferably $\geq 5$, more preferably $\geq 35$, even more preferably $\geq 45$, most preferably $\geq 50$; and/or preferably $\leq 280$, more preferably $\leq 180$, even more preferably $\leq 100$, most preferably $\leq 80$.

14. The mixture according to any one of the preceding aspects, wherein the iodine value of the carboxylic monoacid(s) of formula (II) is 5-280, more preferably 5-180, more preferably 35-180, 35-100, more preferably 45-100, more preferably 45-80, most preferably 50-80.

15. The mixture according to any one of the preceding aspects, wherein the carboxylic monoacid(s) of formula (II) is one or more selected from caproic acid, caprylic acid, 2-ethylhexanoic acid, capric acid, lauric acid, isotridecanoic acid, myristic acid, palmitic acid, palmoleic acid, stearic acid, isostearic acid, oleic acid, elaidic acid, petroselinic acid, linoleic acid, linolenic acid, eleostearic acid, arachic acid, gadoleic acid, behenic acid and erucic acid, and mixtures thereof which are obtained for example by pressure splitting of natural fats and oils, in the reduction of aldehydes from Roelen's oxosynthesis or dimerization of unsaturated fatty acids, stearic acids, isostearic acid, palmitic acid, myristic acid, lauric acid, capric acid, caprylic acid, 2-ethylhexanoic acid, 2-octyldodecanoic acid, capric acid, oleic acid, linoleic acid, linolenic acid, partially hydrogenated coconut fatty acid, palm fatty acid, partially hydrogenated distilled palm fatty acid, hydrogenated distilled palm fatty acid, palm kernel fatty acid, tallow fatty acid, distilled tallow fatty acid, and rapeseed fatty acid.

16. The mixture according to any one of the preceding aspects, wherein the compounds corresponding to a.1 and/or a.2 are from natural origin.

17. The mixture according to any one of the preceding aspects, wherein the compounds corresponding to a.1 and/or a.2 are from synthetic origin.

18. The mixture according to any one of the preceding aspects, wherein Step II corresponds to the formation of the addition salts of the alkanolamine esters obtained from Step I with mineral or organic acids, preferably wherein the mineral or organic acids are one or more selected from hydrochloric, sulphuric, phosphoric, citric and lactic acid.

19. The mixture according to any one of aspects 1-17, wherein Step II corresponds to the quaternisation of reaction mixtures of Step I with alkylating agent(s), preferably wherein the alkylating agents are one or more selected from methyl chloride, methyl bromide, dimethyl sulphate, diethyl sulphate and dimethyl carbonate.

20. The mixture according to any one of the preceding aspects, wherein the polyol a.2 is one or more selected from trimethylolpropane (TMP), glycerine and neopentyl glycol (NPG), each of which can be can be optionally alkoxylated, preferably ethoxylated; wherein the polyol a.2 is more preferably trimethylolpropane (TMP), or is absent.

21. The mixture according to any one of the preceding aspects, further comprising an organic solvent, preferably an alcohol, more preferably ethanol, n-propanol or isopropanol, butanols, glycol, propane or butanediol, glycerol, diglycol, propyl or butyl diglycol, ethylene glycol methyl ether, ethylene glycol ethyl ether, ethylene glycol propyl ether, ethylene glycol mono-n-butyl ether, diethylene glycol methyl ether, diethylene glycol ethyl ether, propylene glycol methyl, ethyl or propyl ether, dipropylene glycol methyl or ethyl ether, methoxy, ethoxy or butoxy triglycol, 1-butoxyethoxy-2-propanol, 3-methyl-3-methoxybutanol, or propylene glycol t-butyl ether.

22. The mixture according to aspect 21, wherein the content of the organic solvent is 0-30%, preferably 0-20%, more preferably 10-20% by weight.

23. The mixture according to any one of the preceding aspects, wherein the mixture is essentially water-free.

24. The mixture according to any one of the preceding aspects, wherein the mixture essentially consists of the reaction products of steps I and II, and optionally an organic solvent.

25. The mixture according to any one of the preceding aspects, wherein the mixture consists of the reaction products of steps I and II and solvent, if any, and unreacted starting materials as well as inevitable impurities from the production process, if any.

26. A softener composition, preferably a fabric-softening and/or keratin-based-fibres-softening composition, comprising the cationic surfactant mixture according to any one of the preceding aspects.

27. The composition according to aspect 26, further comprising water.

28. The composition according to aspect 26 or 27, which is optically transparent at room temperature, and preferably at temperatures of 26-40 °C, more preferably 20-50 °C, even more preferably 16-50 °C, most preferably 5-60 °C.

29. The composition according to any one of aspects 26-28, wherein:

the water content is preferably higher than 50%, more preferably higher than 80%, most preferably higher than 85% w/w; and/or
wherein the solid residue is lower than 50%, more preferably lower than 25%, even more preferably between 15 and 2%;
or wherein:

the water content is 80% or less or 40% or less, preferably 35% or less, more preferably between 35 and 30%, most preferably 30% w/w or less; and/or
the solid residue is 20% or more or 60% or more, preferably 65% or more, more preferably between 65 and 70%, most preferably 70% or more.

30. The composition according to any one of aspects 26-29, further comprising:

A) a perfume, preferably wherein:

(i) the perfume consists of one or more substance(s); and/or
(ii) the average logP of the perfume substance(s) is from 1 to 6; and/or
(iii) the weight ratio between the cationic surfactant mixture and the perfume is from 99:1 to 40:60, more preferably from 80:20 to 60:40, even more preferably 80:20 to 70:30; and/or
(iv) the perfume can be partially encapsulated, preferably the perfume is encapsulated in a biodegradable microcapsule, more preferably the microcapsule is based on chitosan; and/or

B) a non-ionic surfactant, the weight ratio between the cationic surfactant mixture and the non-ionic surfactant is from 100:0 to 70:30; and/or
C) a thickener, wherein the weight ratio of the mixture according to aspect 1 to 25 to the thickener is from 100:0 to 10:5, preferably from 100:1 to 10:2

31. The composition according to 26-30 comprising:

(i) the mixture of cationic surfactants as defined in any one of aspects 1 to 25, and
(ii) optionally one or more non-ionic surfactants,
in which the sum of components (i) and (ii) is from 2% to 100% by weight, wherein,

- the content of component (i) is from 2% to 100%,
- the content of component (ii) is from 0% to 50%, preferably from 0 to 20%; more preferably from 0 to 5%, even more preferably 0 to 2%, most preferably 0 to 0.05% by weight, relative to the weight of the composition.

32. A concentrated composition suitable for preparing a unit dose softener contained in a water-soluble pouch, wherein the composition:

- comprises a mixture according to any one of aspects 1-25, and/or
- is liquid and transparent at a temperature from 26-40 °C, preferably 20-60 °C, more preferably 16-80 °C, most preferably 0-80 °C.

33. The concentrated composition according to aspect 32, which has a water content lower than 10%, preferably lower than 5%, more preferably lower than 1%, even more preferably lower than 0.1%, most preferably 0%.

34. The concentrated composition according to aspect 32 or 33, further comprising:

A) a perfume, preferably wherein:

(i) the perfume consists of one or more substance(s); and/or
(ii) the average logP of the perfume substance(s) is from 1 to 6; and/or

B) a non-ionic surfactant,
which is preferably characterised in that the weight ratio of the described components are values according to the following ratios:

P:C is a value from 0:100 to 60:40
NI:C is 0 or a value from 0:100 to 50:50
P corresponds to the weight content of perfume,
C corresponds to the weight content of cationic surfactant mixture according to any of aspects 1 to 25,
NI corresponds to the weight content of non-ionic surfactant.

35. The concentrated composition according to any one of aspects 32-34, which essentially consists, or consists of, the mixture of cationic surfactants according to any one of aspects 1-35, perfume, optionally non-ionic surfactant, and optionally an organic solvent.

36. A fabric conditioner unit dose comprising:

- a water-soluble pouch acting as container of a liquid composition,
- the fabric softening composition according to aspects 32-35 contained within or by the pouch,
preferably wherein the unit dose is a capsule or a tablet, and preferably wherein the water soluble pouch is

formed of or comprises polyvinyl alcohol.

37. A concentrated softener composition suitable for preparing a domestic softener formulation by dilution, comprising the mixture according to any one of aspects 1-25, and having a water content of 80% or lower, preferably 50% or lower, more preferably 30% or lower, most preferably lower than 5% w/w.

38. The concentrated softener composition according to aspect 37, which is liquid at temperature from 26-40 °C, preferably 20-60 °C, more preferably 16-80 °C, most preferably 0-80 °C

39. The composition of any one of aspects 37-38, which is liquid and transparent at temperature from 26-40 °C, preferably 20-60 °C, more preferably 16-60 °C, most preferably 0-80 °C.

40. The composition according to any one of aspects 37-39, further comprising:

A) a perfume, preferably wherein:

(i) the perfume consists of one or more substance(s); and/or
(ii) the average logP of the perfume substance(s) is from 1 to 6; and/or
(iii) the perfume is an encapsulated perfume, preferably the perfume is encapsulated in a biodegradable microcapsule, more preferably the microcapsule is based on chitosan; and/or

B) a non-ionic surfactant; and/or
C) characterised in that the weight ratio of the described components are values according to the following ratios:

P:C is a value from 0:100 to 60:40NI:C is from 0:100 to 30:70
P corresponds to the perfume, optionally microencapsulated perfume;
C corresponds to the cationic surfactant mixture according to aspects 1 to 25;
NI corresponds to the non-ionic surfactant.

41. The composition according to aspect 30, 31, 34, 35 or 40, wherein the non-ionic surfactant(s) are selected from fatty acids, linear or branched, alkoxylated or non-alkoxylated esters of fatty acids, especially those containing from 8 to 18 carbon atoms, alkoxylated or non-alkoxylated Guerbet alcohols, optionally alkoxylated glycerol and polyglycerol esters, xylitol esters, alkoxylated or non-alkoxylated sorbitan esters, esters of sugars, such as glucose, fructose, galactose, mannose, xylose, arabinose, ribose, 2-deoxyribose and sucrose, $C_{8-18}$ fatty alcohols, alkyl polyglucosides, non-ionic surfactants with amide groups derived from amines, such as glucamine, and the derivatives of methylethanolamine, diethanolamine, isopropanolamine and monoethanolamine, with linear or branched fatty acids, especially those containing from 8 to 18 carbon atoms, waxes, such as paraffins, microcrystalline waxes derived from petroleum, and synthetic waxes, and pentaerythritol esters, especially having a tallow, hydrogenated tallow, palm, behenic or oleic chain, preferably non-ionic surfactants are selected from glycerine esters that are ethoxylated, sorbitan monoesters and pentaerythritol esters, especially those having a tallow, hydrogenated tallow, palm, behenic or oleic chain.

42. The composition according to any one of aspects 26-41, preferably 32-35, characterised in having a viscosity at 20 °C of 200-50,000 cps, as measured on a Brookfield LVT viscometer with spindle 2 at 60 rpm or with spindle 4 at 12 rpm.

43. The composition according to aspect 42, wherein the composition:

A) has a viscosity of 200 to 5,000 mPas as measured on a Brookfield LVT viscometer with spindle 4 at 12 rpm, optionally 200 to 800 mPas; or
B)B) the composition has a viscosity of 200 to 800 mPas as measured on a Brookfield LVT viscometer with spindle 2 at 30 rpm.

44. Use of the mixture of any one of aspects 1-25 for preparing an optically transparent composition; or a process for preparing an optically transparent composition from the mixture of any one of aspects 1-25.

45. The use or process according to aspect 44, comprising combining the mixture with water.

46. Use of the composition of any one of aspects 26-35 and 37-43 for softening fabrics and/or keratin-based-fibres, or a method for softening fabrics and/or keratin-based-fibres comprising a step of contacting the composition of any one of aspects 26-35 and 37-43 with the fabrics and/or fibres.

47. Use of the concentrated composition of any one of aspects 37-43 for softening fabrics and/or keratin-based-fibres comprising preparing a fabric softener comprising mixing the composition with tap water; or a method for preparing a fabric softener; or a method for softening fabrics, each of the methods comprising mixing the composition of any one of aspects 37-43 with tap water.

48. Use of the unit dose of aspect 36 or the composition of any one of aspects 37-43 for softening fabrics and/or fibres, or a method for softening fabrics and/or fibres, comprising introducing the unit dose or the composition directly into a washing machine.

As used herein, "clear" or "optically transparent" appearance of a mixture/composition/formulation refers to essentially complete optical transmittance, e.g., preferably ≥ 90%, more preferably ≥ 95%, even more preferably ≥ 98%, most preferably ≥ 99%. Preferably, and unless specified otherwise, the optical transmittance of such mixture/composition/formulation is measured at $\lambda$ = 600 nm (1 cm thickness, 20 °C).

As used herein, in cases where a ratio (e.g., molar ratio) "x/y" between compound(s) within a first definition "x" and compound(s) under a second definition "y" is 0, this means that compounds within the first definition "x" are absent or essentially absent.

As used herein, and unless specified otherwise, "stable" or "stable upon storage" refer to compositions comprising the cationic surfactant mixture according to the present invention which maintain essentially complete optical transmittance as described above immediately after their preparation and after storage. Preferably, the compositions are stable over at least 7 days at 20 °C; over at least 7 days at 5 °C; over at least 14 d at 20 °C; over at least 28 days at 20 °C; or over at least 28 days at 40 °Cs.

As used herein, viscosity is measured on a Brookfield LVT viscometer at 20 °C with a spindle 2 at 30 or 60 rpm (preferably: for low viscosities), or with a spindle 4 at 12 rpm (preferably: for high viscosities).

As used herein, "iodine number" (or "iodine value", IV) describes the degree of unsaturation, e.g., of a fatty acid, and can be determined according to EN 14111:2003.

## EXAMPLES

### Example 1

***Synthetic procedure***

(i) Esterification:

115.1 grams (0.42 mol) of tallow fatty acid and 202.9 grams (1.39 mol) of Adipic acid were introduced in an inert atmosphere into a glass reactor. Then, 292.3g (1.96 mol) of triethanolamine, together with 65.8 grams of Trimethylolpropane (0.49 mol) were introduced, which were added with stirring. The mixture was heated for at least 4 hours at 160-180°C in order to remove water from the reaction. The final point of the reaction is monitored by an acid value assay until the value was below 2 mg KOH/g.

A yellowish liquid product from the esterification was obtained. consisting essentially of a mixture of unesterified fatty acids and adipic acid, mono-, di- and triesterified triethanolamine with fatty acids, mono-, di- and triesterified triethanolamine with adipic acid or a combination thereof, mono-, di- and triesterified trimethylolpropane with fatty acids, mono-, di- and triesterified trimethylolpropane with adipic acid or a combination thereof, together with unreacted triethanolamine and trimethylolpropane.

(ii) Quaternisation:

611.5 grams of the product from esterification step (containing 1.94 mol of amine equivalent product) are mixed with 149.0 g of Ethanol (3.23 mol), then, 232.4 grams (1.84 mol) of dimethyl sulphate were added with stirring at a temperature of 50-90°C.

After four hours of digestion, the virtually complete absence of amine value was verified by acid/base assay. 992.9 grams of the final product was obtained.

***Calculations***

Molar ratio monocarboxylic acid / dicarboxylic acid:

$$0.42 \text{ mol tallow fatty acid} / 1.39 \text{ mol adipic acid} = 0.30$$

Equivalents ratio COOH / OH:

.

$$(1 \text{ eq} * 0.42 \text{ mol tallow fatty acid} + 2 \text{ eq} * 1.39 \text{ mol adipic acid}) / (3 \text{ eq} * 1.96 \text{ mol triethanolamine} + 3 \text{ eq} * 0.49 \text{ mol trimethylolpropane}) = 0.44$$

Molar ratio polyol / triethanolamine:

$$0.49 \text{ mol trimethylolpropane} / 1.96 \text{ mol triethanolamine} = 0.25$$

**Example 2**

A quaternary esterammonium-containing composition according to the present invention (EQ) was mixed with perfume at room temperature at different ratios. The resulting mixtures were optically transparent. To quantify the optically transparent appearance, transmittance was measured at $\lambda$ = 600 nm (1 cm thickness, 20 °C) **(Table 1).** Viscosities were measured with Spindle 2, 60 rpm (viscosity <500 mPa·s); Spindle 2, 6 rpm (viscosity < 5000 mPa·s); or Spindle 4, 30 rpm (viscosity > 5000 mPa·s).

The clear viscous liquid that can be introduced directly to the washing machine as a tablet or a concentrated product.

**Table 1:** Exemplary EQ/perfume mixtures (parts by weight), and properties thereof

| Sample | 1 | 2 | 3 | 4 | 5 |
|---|---|---|---|---|---|
| 25733 | 80 | 60 | 40 | 70 | 40 |
| FINDET 1315/19 | | | | 10 | |
| Propyleneglycol | | | | | 50 |
| Fragrance | 20 | 40 | 60 | 20 | 10 |
| | | | | | |
| Transmittance (%) | >95 | >95 | >95 | >95 | >95 |
| Viscosity | 2970 | 1168 | 350 | 2599 | 260 |

EQ composition 25733 is a quaternary esterammonium-containing composition prepared in a manner analogous to that in Example 1, wherein step (i), the molar ratio between organic carboxylic groups and organic hydroxyl groups present in the system of was 0.53, no polyol was present, the monoacid was PH Palm fatty acid, and the diacid was adipic acid.

FINDET 1315/19: $C_{13-15}$ alcohol 7EO, available from KAO Chemicals Europe.

Fragrance: commercial standard fabric softener fragrance for blue line products, available from KAO Chemicals Europe.

**Example 3**

The following EQ compositions **(Table 2)** were prepared by an analogous procedure as in Example 1.

70 g of each EQ composition were mixed with 30 g of fragrance at room temperature.

The resulting mixtures with compositions according to the invention were clear viscous liquids. The clear viscous liquid that can be introduced directly to the washing machine as a tablet or a concentrated product.

**Table** 2: Exemplary EQ compositions

| **EQ composition** | **25105** | **25115** | **25471** | **25625** | **25153** | **25267** | **T.AO-1** | **T.L6-90E** |
|---|---|---|---|---|---|---|---|---|
| Molar ratio monocar-boxylic acid/dicarboxylic acid in step (i) | 0.3 | 0.6 | 1.20 | 1.5 | 3.5 | 10 | - | - |
| Equivalents ratio COOH/OH in step (i) | 0.6 | 0.5 | 0.53 | 0.3 | 0.6 | 0.6 | 0.57 | 0.55 |
| Molar ratio polyol/TEA in step (i) | 0.14 | 0.33 | 0.14 | 0 | 0.25 | 0.14 | - | - |
| Polyol used in step (i) | TMP | Glycerine | TMP | - | TMP | TMP | - | - |
| Diacid used in step (i) | Adipic | Adipic | Suberic | Suberic | Adipic | Adipic | - | - |
| Monoacid used in step (i) | TLH-D | Palm PH | Palm PH | TLH-D | TLH-D | Palm PH | Oleic Acid | Palm PH |
| Solvent | Ethanol | Ethanol | Ethanol | Ethanol | Ethanol | Ethanol | IPA | EtOH |
| | | | | | | | | |
| Appearance at 20°C 70% EQ + 30% perfume | Clear viscous liquid | Clear viscous liquid | Clear viscous liquid | Clear viscous liquid | Clear viscous liquid | Turbid viscous liquid | Clear viscous liquid | Separated paste |
| TLH-D: Distilled tallow fatty acid / Edenor® Ti E GA, CYPACID TL-12<br>Palm PH: Partially hydrogenated distilled palm fatty acid / CYPACID P-50-H, Radiacid® 0438<br>Perfume: same as above | | | | | | | | |

**Example 4**

70 g of an EQ composition of the present invention were mixed with 30 g of fragrance at room temperature. 6 g of the mixture was added inside a bottle, and 100 mL of tap water (20°HF) were added for dilution. The bottle was shaken gently to obtain the liquid fabric softener. Complete dispersion was observed with the compositions according to the invention **(Table 3).** In contrast, the comparative sample resulted in an opaque, pasty, viscous, mucus-like product.

The results show that the compositions of the invention allow to produce highly concentrated fabric softener compositions comprising EQ in combination with perfume, which can be used to prepare a fabric softener at home using tap water.

**Table** 3: Dispersion and appearance performance of EQ/perfume compositions

| **Sample** | **6** | **7** | **8** | **9** | **10** | **11** |
|---|---|---|---|---|---|---|
| EQ composition | 25105 | 25115 | 25471 | 25625 | 25153 | T.AO-1 |
| Dispersion time in water 20°HF (EQ+perfume) (s) | 300 | 14 | 8 | 16 | 5 | 60 |
| Appearance of dispersion | Clear-Turbid | Clear-Turbid | Turbid | Turbid | Opaque | Opaque, pasty, viscous, mucus-like |
| Perfume: same as above | | | | | | |

**Claims**

1. A mixture of cationic surfactants obtainable from a process comprising the steps:

Step I: esterification of a) with b), and
Step II: cation formation from the reaction products of Step I,
wherein:

a) is a hydroxyl group-containing compound or a mixture of hydroxyl group-containing compounds comprising a.1 and optionally a.2, wherein:

- a.1 is an alkanolamine or a mixture of alkanolamines of the general formula (I):

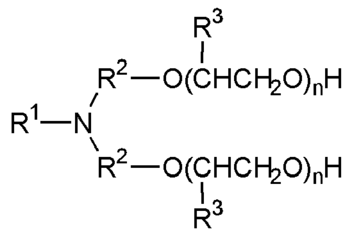

**(I)**

in which $R^1$ is selected from hydrogen, a $C_1$-$C_6$ alkyl group, and the residue

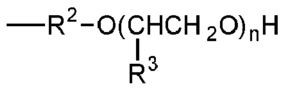

,

$R^2$ is a $C_1$-$C_6$ alkylene group, $R^3$ is hydrogen or methyl, n is 0 or an integer from 1 to 20; and
- a.2 is a polyol, which can be optionally alkoxylated, and is **characterized by** a MW in the range 60 to 190 g/mol;

b) is a mixture of compounds containing one or more carboxylic groups comprising b.1 and b.2, wherein:

- b.1 is a monocarboxylic acid or a mixture of monocarboxylic acids of formula (II):

$R^6$-COOH (II)

in which $R^6$ is a linear or branched $C_6$-$C_{23}$ alkyl or alkenyl group; or an alkyl ester or glyceride thereof, preferably a linear or branched $C_6$-$C_{23}$ alkyl or alkenyl ester; and
- b.2 is a dicarboxylic acid or a mixture dicarboxylic acids of the general formula (III), or reactive derivative(s) thereof:

HOOC-L-COOH (III)

wherein L is a saturated or unsaturated, linear, branched or cyclic group having 1 to 10 carbon atoms, each of which carbon atoms is optionally substituted by a $C_1$-$C_6$ saturated or unsaturated group; and is preferably represented by $(CH(R^7))_m$ or by ($C_6$-$C_{10}$ arylene) optionally substituted by one or more $R^7$, in which each $R^7$ is independently a hydrogen, OH or a $C_1$-$C_6$ saturated or unsaturated group, m is 0 or an integer from 1 to 10, wherein for $m \geq 2$, the chain $(CH)_m$ optionally contains one or more double bonds and/or cyclic group(s);

wherein a.1), a.2), b.1) and b.2) are introduced in the reaction system of Step I in amounts resulting in the following molar ratios:

- the molar ratio of monoacid(s)/diacid(s) (b.1/b.2) is 0.10 to 5.0;
- the molar ratio between organic carboxylic groups and organic hydroxyl groups (COOH/OH) present in the

system is 0.30 to 0.70; and
- the molar ratio between the compound(s) within the definition a.2 and the compound/s under definition a.1 is 0 to 0.5.

**2.** The mixture according to claim 1, wherein the amounts of the compounds a.1, a.2, b.1 and b.2 are introduced in the reaction system of Step I in amounts that result in the following molar ratios:

- the molar ratio of monoacid(s)/diacid(s) (b.1/b.2) is 0.5 to 3.5;
- the molar ratio between organic carboxylic groups and organic hydroxyl groups (COOH/OH) present in the system is 0.40 to 0.70; and
- the molar ratio between the compound(s) within the definition a.2 and the compound/s under definition a.1 is 0, or is 0.1 to 0.5.

**3.** The mixture according to claim 1 or 2, wherein:

A) the alkanolamines(s) of formula (I) is selected from triethanolamine, N-methyldiethanolamine, N-methyldiisopropanolamine and triisopropanolamine, each of which is optionally alkoxylated with ethylene oxide or propylene oxide, and mixtures thereof; and/or
B) the dicarboxylic acid(s) of formula (III) is selected from succinic, malic, glutaric, adipic, sebacic, pimelic, suberic, maleic and terephthalic acid, acids obtained by thermal oligomerisation of unsaturated fatty acids, and mixtures thereof; and/or
C) the monocarboxylic acid(s) of formula (II) are synthetic fatty acids or are obtained from fats or oils of natural origin and are optionally hydrogenated, preferably from oils of vegetal origin which are optionally hydrogenated, preferably wherein the monocarboxylic acids of formula (II) are selected from those which are obtained from tallow, palm, olive, coconut, sunflower, soya, rapeseed, grape marc and grape, each of which can be hydrogenated, partially hydrogenated, or non-hydrogenated; and/or
D) the compounds corresponding to a.1 and/or a.2 are from natural origin.

**4.** The mixture according to any one of the preceding claims, wherein:

A) Step II corresponds to the formation of the addition salts of the alkanolamine esters obtained from Step I with mineral or organic acids,
preferably wherein the mineral or organic acids are one or more selected from hydrochloric, sulphuric, phosphoric, citric and lactic acid; or
B) Step II corresponds to the quaternisation of reaction mixtures of Step I with alkylating agent(s),
preferably wherein the alkylating agents are one or more selected from methyl chloride, methyl bromide, dimethyl sulphate, diethyl sulphate and dimethyl carbonate.

**5.** A softener composition, preferably fabric-softening and/or keratin-based-fibres-softening composition, comprising the mixture according to any one of the preceding claims.

**6.** The composition according to claim 5, further comprising:

A) a perfume, preferably wherein:

(i) the perfume consists of one or more substance(s); and/or
(ii) the average logP of the perfume substance(s) is from 1 to 6; and/or
(iii) the weight ratio between the cationic surfactant mixture and the perfume is from 99:1 to 40:60, more preferably from 80:20 to 60:40, even more preferably 80:20 to 70:30; and/or
(iv) the perfume can be partially encapsulated, preferably the perfume is encapsulated in a biodegradable microcapsule, more preferably the microcapsule is based on chitosan; and/or

B) a non-ionic surfactant, the weight ratio between the cationic surfactant mixture and the non-ionic surfactant is from 100:0 to 70:30; and/or
C) a thickener, wherein the weight ratio of the mixture according to claim 1 to 5 to the thickener is from 100:0 to 10:5, preferably from 100:1 to 10:2

**7.** The composition according to any one of claims 5 to 6 comprising:

(i) the mixture of cationic surfactants as defined in any one of claims 1 to 4, and
(ii) optionally one or more non-ionic surfactants,
in which the sum of components (i) and (ii) is from 2% to 100% by weight, wherein,

- the content of component (i) is from 2% to 100%,
- the content of component (ii) is from 0% to 50%, preferably from 0 to 20%; more preferably from 0 to 5%, even more preferably 0 to 2%, most preferably 0 to 0.05% by weight, relative to the weight of the composition.

**8.** A composition suitable for preparing a unit dose softener contained in a water-soluble pouch, wherein the composition:

- comprises a mixture according to claims 1 to 4, and
- is liquid and transparent at a temperature from 26-40 °C, preferably 20-60 °C, more preferably 16-80 °C, most preferably 0-80 °C, and

which preferably has a water content lower than 10%, preferably lower than 5%, preferably lower than 1%, preferably lower than 0.1%, most preferably 0%.

**9.** The composition according to claim 8, further comprising:

A) a perfume, preferably wherein:

(i) the perfume consists of one or more substance(s); and/or
(ii) the average logP of the perfume substance(s) is from 1 to 6;
and/or

B) a non-ionic surfactant,
which is preferably **characterised in that** the weight ratio of the described components are values according to the following ratios:

P:C is a value from 0:100 to 60:40
NI:C is 0 or a value from 0:100 to 50:50
P corresponds to the weight content of perfume,
C corresponds to the weight content of cationic surfactant mixture according to claims 1 to 4,
NI corresponds to the weight content of non-ionic surfactant.

**10.** A fabric conditioner unit dose comprising:

- a water-soluble pouch acting as container of a liquid composition,
- the fabric softening composition according to claim 8 or 9 contained within or by the pouch;

preferably wherein the unit dose is a capsule or a tablet, and/or preferably wherein the water soluble pouch is formed of or comprises polyvinyl alcohol.

**11.** A concentrated softener composition suitable for preparing a domestic softener formulation by dilution **characterised by** the following features:

- the composition is liquid at temperature from 26-40 °C, preferably 20-60 °C, more preferably 16-80 °C, most preferably 0-80 °C, and
- the composition comprises a product according to claims 1 to 4, and
- the composition has a water content of 80% or lower, preferably 50% or lower, more preferably 30% or lower, most preferably lower than 5% w/w.

**12.** The composition according to claim 11, further comprising:

A) a perfume, preferably wherein:

(i) the perfume consists of one or more substance(s); and/or
(ii) the average logP of the perfume substance(s) is from 1 to 6; and/or
(iii) the perfume can be an encapsulated perfume, preferably the perfume is encapsulated in a biodegradable microcapsule, more preferably the microcapsule is based on chitosan; and/or

B) a non-ionic surfactant; and/or

C) **characterised in that** the weight ratio of the described components are values according to the following ratios:

P:C is a value from 0:100 to 60:40 NI:C is from 0:100 to 30:70
P corresponds to the perfume, optionally microencapsulated perfume
C corresponds to the cationic surfactant composition according to claims 1 to 4
NI corresponds to the non-ionic surfactant.

**13.** The composition according to any one of claims 5-9, 11 and 12, **characterised in** having a viscosity at 20 °C of 200-50000 cps, as measured with spindle 2 at 60 rpm or with spindle 4 at 12 rpm, preferably wherein:

A) the composition has a viscosity of 200 to 5000 mPas as measured on a Brookfield LVT viscometer with spindle 4 at 12 rpm, optionally 200 to 800 mPas; or

B) the composition has a viscosity of 200 to 800 mPas as measured on a Brookfield LVT viscometer with spindle 2 at 30 rpm.

**14.** Use of the composition of any one of claims 5-9, 12 and 131, or the unit dose of claim 10, for softening fabrics and/or keratin-based-fibres.

**15.** Use of the composition of any one of claims 11-13 for softening fabrics and/or keratin-based-fibres comprising preparing a fabric softener comprising mixing the composition with tap water; or
of the unit dose of claim 10 or the composition of any one of claims 11-13 for softening fabrics and/or fibres, comprising introducing the unit dose or the composition directly into a washing machine.

Europäisches Patentamt
European Patent Office
Office européen des brevets

# EUROPEAN SEARCH REPORT

Application Number
EP 22 38 2185

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 136 471 A1 (KAO CORP SA [ES]) 26 September 2001 (2001-09-26) * paragraphs [0013] - [0025], [0028], [0030], [0031], [0043], [0045], [0048] - [0051]; tables 1,2 * | 1-15 | INV. C11D1/62 C11D1/835 C11D3/00 C11D3/50 C11D11/00 A61Q5/12 C07C209/20 C11D17/04 ADD. C11D1/74 |
| X | US 2003/130162 A1 (LLOSAS JOAQUIM BIGORRA [ES] ET AL) 10 July 2003 (2003-07-10) * paragraphs [0005] - [0008], [0010] - [0028], [0080] - [0084], [0127] * | 1-15 | |
| X | US 2002/002297 A1 (KEYS ROBERT O [US]) 3 January 2002 (2002-01-03) * paragraphs [0008], [0013], [0016], [0026] - [0044], [0047], [0127]; claims 1,5,10,13 * | 1-15 | |
| A | US 2004/142840 A1 (DE BUZZACCARINI FRANCESCO [BE] ET AL) 22 July 2004 (2004-07-22) * paragraphs [0011] - [0018] * | 1,10,12 | TECHNICAL FIELDS SEARCHED (IPC) |
| A | US 2006/135399 A1 (GRANDMAIRE JEAN-PAUL [BE] ET AL) 22 June 2006 (2006-06-22) * paragraphs [0012], [0013], [0018], [0024] - [0027] * | 1-15 | C11D C07C A61Q |

The present search report has been drawn up for all claims

1

| Place of search | Date of completion of the search | Examiner |
|---|---|---|
| The Hague | 12 August 2022 | Goodman, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 22 38 2185

This annex lists the patent family members relating to the patent documents cited in the above-mentioned European search report.
The members are as contained in the European Patent Office EDP file on
The European Patent Office is in no way liable for these particulars which are merely given for the purpose of information.

12-08-2022

| Patent document cited in search report | Publication date | Patent family member(s) | Publication date |
|---|---|---|---|
| EP 1136471 A1 | 26-09-2001 | AT 245139 T | 15-08-2003 |
| | | DE 60100461 T2 | 15-04-2004 |
| | | EP 1136471 A1 | 26-09-2001 |
| | | ES 2180372 A1 | 01-02-2003 |
| | | ES 2198392 T3 | 01-02-2004 |
| | | JP 4021152 B2 | 12-12-2007 |
| | | JP 2001335545 A | 04-12-2001 |
| | | PT 1136471 E | 31-10-2003 |
| | | US 6465419 B1 | 15-10-2002 |
| US 2003130162 A1 | 10-07-2003 | AU 1864101 A | 09-07-2001 |
| | | DE 19962874 A1 | 28-06-2001 |
| | | EP 1239827 A1 | 18-09-2002 |
| | | ES 2231291 T3 | 16-05-2005 |
| | | JP 2003519294 A | 17-06-2003 |
| | | US 2003130162 A1 | 10-07-2003 |
| | | WO 0147489 A1 | 05-07-2001 |
| US 2002002297 A1 | 03-01-2002 | CA 2349414 A1 | 01-12-2001 |
| | | EP 1160238 A1 | 05-12-2001 |
| | | US 2002002297 A1 | 03-01-2002 |
| US 2004142840 A1 | 22-07-2004 | AU 2003297479 A1 | 14-07-2004 |
| | | BR 0317415 A | 08-11-2005 |
| | | CA 2505535 A1 | 08-07-2004 |
| | | EP 1431383 A1 | 23-06-2004 |
| | | JP 2006520823 A | 14-09-2006 |
| | | MX PA05006590 A | 16-08-2005 |
| | | US 2004142840 A1 | 22-07-2004 |
| | | WO 2004056958 A1 | 08-07-2004 |
| US 2006135399 A1 | 22-06-2006 | US 2006135399 A1 | 22-06-2006 |
| | | WO 2005073358 A1 | 11-08-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016096614 A1